# EUROPÄISCHE PATENTSCHRIFT

(11) **EP 2 265 939 B1**
(45) Veröffentlichungstag und Bekanntmachung des Hinweises auf die Patenterteilung: **21.08.2013**
(21) Anmeldenummer: 09730576.7
(22) Anmeldetag: 21.03.2009
(51) Int. Cl.: G01N 21/85, G01N 33/14, G01N 33/00

(54) **VERFAHREN SOWIE MESSEINRICHTUNG ZUR BESTIMMUNG DES GEHALTS AN WENIGSTENS EINEM FILTERHILFSMITTEL IN EINEM FLÜSSIGEN MEDIUM**
METHOD AND MEASURING DEVICE FOR DETERMINING THE CONTENT IN AT LEAST ONE FILTER AID IN A LIQUID MEDIUM
PROCÉDÉ ET DISPOSITIF DE MESURE POUR DÉTERMINER LA TENEUR D'UN MILIEU LIQUIDE EN AU MOINS UN ADJUVANT DE FILTRAGE

(30) Priorität: 09.04.2008 DE 102008018102
(43) Veröffentlichungstag der Anmeldung: 29.12.2010
(73) Patentinhaber: KHS GmbH, 44143 Dortmund (DE)
(72) Erfinder: WESTNER, Hans, 55595 Mandel (DE); STIENEN, Thomas, 59425 Unna (DE)
(86) Internationale Anmeldenummer: PCT/EP2009/002100
(87) Internationale Veröffentlichungsnummer: WO 2009/124649

(56) Entgegenhaltungen:
- CH-A- 503 319
- DE-A1- 19 540 456
- DE-A1- 19 612 313
- US-A1- 2006 073 077

## Beschreibung

Die Erfindung bezieht sich auf ein Verfahren sowie auf eine Vorrichtung zur Bestimmung des Gehalts an wenigstens einem Filterhilfsmittel in einem flüssigen Medium, beispielsweise in einem Unfiltrat gemäß Oberbegriff Patentanspruch 1 bzw. 8.

Bei der Filtration von flüssigen Medien oder Flüssigkeiten, beispielsweise bei der Filtration von Getränken, z.B. Bier, kommen in der Regel Filter, z.B. auch Kesselfilter mit Filterkerzen zum Einsatz, die (Filter) die Zugabe eines Filterhilfsmittel, z.B. Kieselgur, erforderlich machen, um das gewünschte Filtrationsergebnis zu erreichen.

Das Filterhilfsmittel, welches dem Unfiltrat beigemischt wird, lagert sich an einem einen Unfiltretraum von einem Filtratraum des Filters trennenden Filterelement, beispielsweise an der Außenfläche der Filterkerzen an und bildet dort eine Filterschicht, welche die aus dem Unfiltrat auszufilternden Stoffe, wie Trübstoffe usw. aufnimmt

Für ein optimales Filtrationsergebnis ist es erforderlich, dem zu filtrierenden Medium oder Produkt je Einheit (Menge, Masse oder Volumen) eine bestimmte Menge an Filterhilfsmittel zuzusetzen. Hierbei ist auch zu beachten, dass durch die geometrischen Abmessung des jeweiligen Filter, beispielsweise durch die geometrischen Abmessungen des Kesselfilters, der Filterkerzen, des Abstandes, den die Filterkerzen voneinander aufweisen, usw. nur eine bestimmte Maximalmenge an Filterhilfsmittel im betreffenden Filter aufgenommen werden kann. Nach Erreichen dieser Maximalmenge ist ein Reinigen und/oder Rückspülen des betreffenden Filters mit Unterbrechung der normalen Produktion erforderlich.

Um einerseits das optimale Filtrationsergebnis zu erreichen und andererseits die Zeiten zwischen zwei Reinigungs- und Rückspülvorgängen möglichst groß zu halten, wird angestrebt, die Zugabe bzw. das Beimischen des Filterhilfsmittels an das zu filtrierende Medium oder Unfiltrat möglichst genau zu dosieren. Was bisher insbesondere auch automatisiert nicht oder nur mit sehr unbefriedigendem Ergebnis möglich ist.

Üblicherweise wird das Filterhilfsmittel dem Unfiltrat als Suspension zugeführt, welche das Filterhilfsmittel in einer Flüssigkeit, beispielsweise in Wasser oder aber in dem zu filtrierenden Medium enthält. Die Herstellung der Suspension erfolgt dann beispielsweise mittels eines Rührwerkes, mit dem das Filterhilfsmittel mit der Flüssigkeit vermischt und zu der Suspension aufgeschlämmt wird.

Bei bekannten Verfahren zur Erzeugung der Suspension sind allerdings ständige manuelle Eingriffe durch das Bedienungspersonal erforderlich, was u.a. dazu führt, dass die Zusammensetzung der Suspension, d.h. der Anteil des Filterhilfsmittels in der Suspension nicht mit der erforderlichen Genauigkeit bekannt ist. Dieses hat zur Folge, dass ein einfaches mengen- und/oder volumengesteuertes Zumischen der Suspension zu dem zu filtrierenden Medium oder Unfiltrat nicht zu einer Dosierung des Füterhilfsmittels mit einer ausreichenden Genauigkeit führt.

Hinzu kommt, dass die für das Beimischen der Suspension übilcherweise verwendeten Dosierpumpen erhebliche Ungenauigkeiten aufweisen, so dass insgesamt gesehen bisher eine automatisierte Dosierung des Filterhilfsmittels mit einer zufrieden stellenden Genauigkeit nicht möglich ist.

Bekannt ist weiterhin, den Filtratstrom, also das flüssige Medium nach der Filtration auf das Vorhandensein von Trübstoffen, beispielsweise auch auf das Vorhandensein von Filterhilfsmittel zu untersuchen, und zwar beispielsweise mit einer Streulicht-Trübungsmessung oder durch eine Absorptionsmessung. Mit diesen bekannten Verfahren ist eine grobe Ermittlung oder Anzeige von Konzentrationsänderungen u.a. des Filterhilfsmittels im Filtrat möglich, keinesfalls aber eine Ermittlung und/oder Anzeige des tatsächlichen Gehalts des Filterhilfsmittels im Filtrat. Mit dem bekannten Verfahren ist es insbesondere auch nicht möglich, die Menge des dem Unfiltrat und damit auch dem jeweiligen Filter zugeführten Filterhilfsmittels zu bestimmen und gar automatisiert zu steuern, zu regeln oder zu begrenzen. Dabei ist insbesondere die Bestimmung der Menge des einem Filter insgesamt zugeführten Filterhilfsmittels von Bedeutung, da jeder Filter nur eine bestimmte Menge von Filterhilfsmitteln aufnehmen kann, und da eine Überschreitung dieser Menge, das so genannte Überladen eines Filters, in der Regel zu einer Beschädigung des Filters führt.

Bekannt ist auch die Zugabe von Filterhilfsmitteln zu einem flüssiges Medium (Unfiltrat), beispielsweise die Zugabe von Füterhilfsmitteln zu Bier in Abhängigkeit von einem elektrischen Messsignal einer opto-elektrischen Messstrecke zu steuern, mit der die Trübung des mit dem Filterhilfsmittel versetzten Mediums erfasst wird (CH 503 319 A1).

Aufgabe der Erfindung ist es, ein Verfahren aufzuzeigen, mit dem das Bestimmen bzw. Messen das Gehalts an dem wenigstens einen Filterhilfsmittel in einem flüssigen Medium oder Unfiltrat mit hoher Genauigkeit möglich ist. Zur Lösung dieser Aufgabe ist ein Verfahren entsprechend dem Patentanspruch 1 ausgebildet. Eine Vorrichtung zum Bestimmen bzw. Messen des Gehalts an wenigstens einem Filterhilfsmittel in einem flüssigen Medium oder Unfiltrat ist Gegenstand des Patentanspruches 8.

Durch die Verwendung von wenigstens zwei Messstellen, die räumlich voneinander getrennt angeordnet sind und von denen jede wenigstens einen Messwert liefert, der zumindest einer sich mit dem Vorhandensein des wenigstens einen Filterhilfsmittels ändernden chemischen und/oder physikalischen Eigenschaften des flüssigen Mediums entspricht, ist durch Vergleich der Messwerte dieser Messstellen eine sehr genaue Bestimmung des Gehalts bzw. des Anteils an dem wenigstens einem Filterhilfsmittel in dem flüssigen Medium möglich, und zwar insbesondere auch dadurch, dass den jeweiligen Messwert fälschende oder beeinflussende Einflussgrößen, auch des flüssigen Mediums oder Änderungen solcher Einflussgrößen durch die Verwendung von wenigstens zwei Messwerten kompensiert werden.

Derartige, nicht durch das Vorhandensein oder Fehlen des Filterhilfsmittels bedingte Einflußgrößen sind z.B. die Temperatur, die für die Jeweilig Messung zur Verfügung stehende Zeit, der Anteil von nicht gelöstem bzw. ausgegastem CO2 im flüssigen Medium usw. sowie die Änderungen dieser Einflussgrößen.

Die von den Messstellen erfassten chemischen und/oder physikalischen Eigenschaften können bei entsprechender Ausbildung und Anpassung der Messstellen unterschiedlichster Art sein, beispielsweise aber nicht beschränkend elektrische Eigenschaften des flüssigen Mediums, wie Leitfähigkeit, Kapazität, mechanische und strömungsmäßige Eigenschaften des flüssigen Mediums, wie Dichte, Viskosität, Druck, Staudruck, Absolutdruck, Differenzdruck, auch Druckabfall an einer Drosselblende, optische Eigenschaften des flüssigen Mediums, wie Reflektionsvermögen, Transparenz, chemische Eigenschaften des flüssigen Mediums, wie pH-Wert usw. Zur Messung dieser Eigenschaften sind die Messstellen entsprechend angepasst.

Zur Erhöhung der Genauigkeit kann es zweckmäßig sein, dass zumindest eine der Messstellen, bevorzugt aber sämtliche Messstellen für das Erfassen von unterschiedlichen chemischen und/oder physikalischen Eigenschaften ausgebildet sind, wobei dann die diesen Eigenschaften entsprechenden Messwerte entweder jeweils parallel, d.h. zeitgleich oder im Wesentlichen zeitgleich oder aber seriell, d.h. zeitlich nacheinander bereit gestellt werden.

Weiterbildungen, Vorteile und Anwendungsmöglichkeiten der Erfindung ergeben sich auch aus der nachfolgenden Beschreibung von Ausführungsbeispielen und aus den Figuren. Dabei sind alle beschriebenen und/oder bildlich dargestellten Merkmale für sich oder in beliebiger Kombination grundsätzlich Gegenstand der Erfindung, unabhängig von ihrer Zusammenfassung in den Ansprüchen oder deren Rückbeziehung. Auch wird der Inhalt der Ansprüche zu einem Bestandteil der Beschreibung gemacht.

Die Erfindung wird im Folgenden anhand der Figuren an einem Ausführungsbeispiel näher erläutert. Es zeigen:

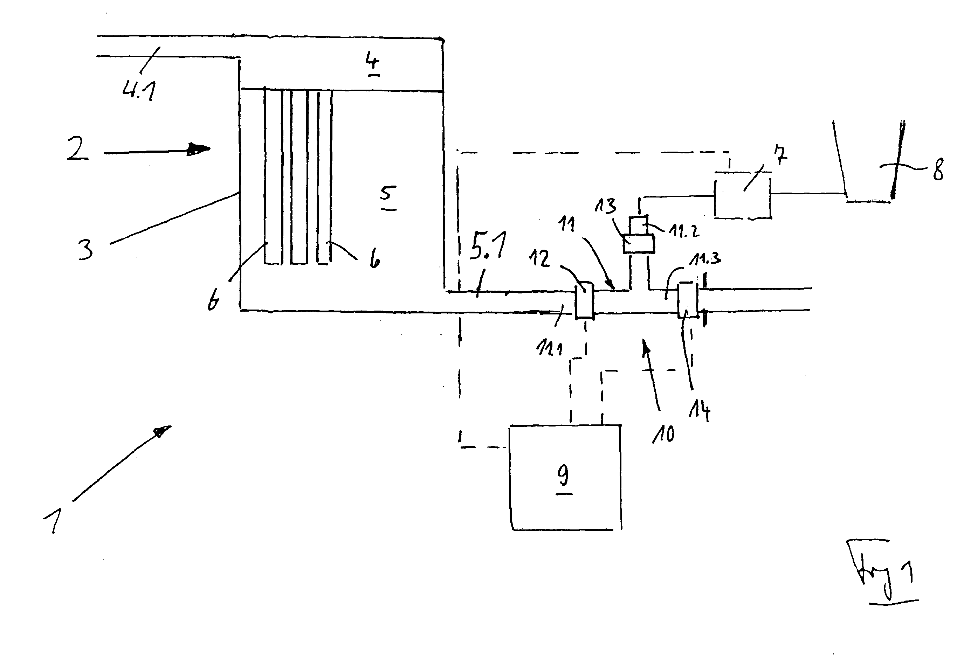

- Fig. 1: eine Anlage zum Filtrieren von flüssigen Medien, beispielsweise zum Filtrieren von Getränken, beispielsweise Bier, zusammen mit einer Einrichtung zur dosierten Zugabe eines Filterhilfsmittels;

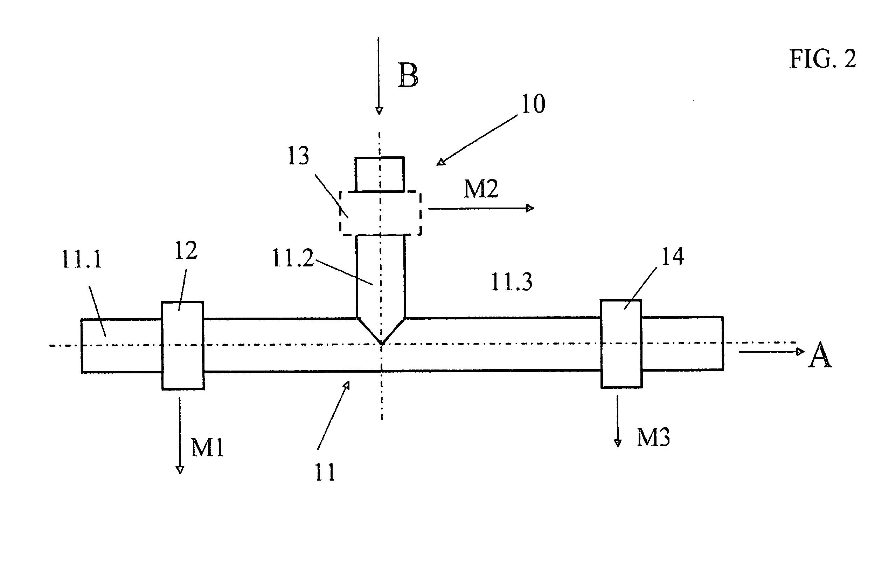

- Fig. 2: in vereinfachter Darstellung eine Messeinrichtung zur Ermittlung des Anteils an Filterhilfsmittel in dem den Filter zugeführten Unfiltrat;
- Fig. 3: in schematischer Darstellung den zeitlichen Verlauf eines Messwertes M an einer der Messstellen der Messeinrichtung der Figuren 1 und 2.

Die in den Figuren allgemein mit 1 bezeichnete Anlage dient zum Filtrieren eines flüssigen Mediums bzw. Unfiltrats, beispielsweise zum Filtrieren von Getränken, auch CO2-haltigen Getränken, z.B. von Bier. Die Anlage 1 ist hierfür mit einem Filter 2 ausgebildet, welches bei der dargestellten Ausführungsform ein Kerzenfilter üblicher Bauart ist, und zwar im Wesentlichen bestehend aus einem in einem Kessel oder Filtergehäuse 3 gebildeten Unfiltratraum 5, dem das Unfiltrat an einem Einlass 5.1 zugeführt wird, aus einem ebenfalls im Filtergehäuse 3 gebildeten Filtratraum 4 mit Auslass 4.1 zum Abführen des filtrierten Mediums bzw. Filtrats sowie aus mehreren strumpfartigen Filterkerzen 6 aus einem für derartige Filterelemente geeigneten Material, die zum Filtratraum 4 hin offen sind und zum Unfiltratraum 5 geschlossenwandig ausgebildet sind.

Um das gewünschte Filterergebnis zu erreichen, wird dem Unfiltrat, welches über den Einlass 5.1 zugeführt wird, wenigstens ein Filterhilfsmittel zugesetzt, und zwar in Form einer das Filterhilfsmittel enthaltenen Suspension, beispielsweise in Form einer Suspension aus dem Unfiltrat und dem Filterhilfsmittel, oder aber auch in Form einer Suspension aus Wasser und dem Filterhilfsmittel.

Als Filterhilfsmittel eignet sich beispielsweise Kieselgur, wobei die Erfindung selbstverständlich nicht auf die Verwendung dieses speziellen Filterhilfsmittels beschränkt ist, sondern auch andere geeignete Filterhilfsmittel Verwendung finden können.

Um das angestrebte Filterergebnis zu erreichen, ist es erforderlich, dem zu filtrierten Medium eine ausreichende Menge an Filterhilfsmittel beizumischen. Da aber die Maximalmenge an Filterhilfsmittel, die (Maximalmenge) von dem Filter 2 bzw. von den Filterkerzen 6 aufgenommen werden kann, insbesondere durch die geometrischen Abmessungen des Unfiltratraumes 4 und der Filterkerzen 6 sowie durch den Abstand der Filterkerzen 6 vorgegeben ist und beispielsweise maximal 300 kg beträgt und da nach Erreichen dieser Maximalmenge jeweils ein Reinigen und/oder Rückspülen des Filters 2 bei Unterbrechung der normalen Produkten erforderlich ist, ist es notwendig, das Filterhilfsmittel bzw. die das Filterhilfsmittel enthaltende Suspension möglichst genau dosiert zuzuführen.

Dies erfolgt über eine Dosiereinrichtung 7, die beispielsweise eine Dosierpumpe und mit einer Einrichtung 8 zur Bereitstellung der Suspension verbunden ist. Angesteuert wird die Dosiereinrichtung 7 von einer Steuereinrichtung 9 in Abhängigkeit von Messsignalen oder Messwerten M1 - M3, die mit einer Messeinrichtung 10 ermittelt werden.

Die Messeinrichtung 10 besteht bei der dargestellten Ausführungsform aus einem dem Einlass 4.1 vorgeschalteten T-förmigen Rohrstück 11 mit drei Rohrabschnitten 11.1 - 11.3, von denen der Rohrabschnitt 11.3 an eine Leitung zum Zuführen des Unfiltrats, der Rohrabschnitt 11.2 mit der Dosiereinrichtung 7 und der Rohrabschnitt 11.1 mit dem Einlass 5.1 verbunden sind.

Die Messeinrichtung 10 weist bei der dargestellten Ausführungsform weiterhin drei Messstellen 12 - 14 auf, und zwar die Messstelle 12 an dem Rohrabschnitt 11.1, die Messstelle 13 an dem Rohrabschnitt 11.2 und die Messstelle 14 an dem Rohrabschnitt 11.3. Die Messstellen 12 - 13 sind so ausgebildet, dass sie jeweils wenigstens einen Messwert M1 - M3 liefern, der wenigstens einem chemischen und/oder physikalischen Wert oder einer chemischen und/oder physikalischen Eigenschaft des die jeweilige Messstelle 12 - 14 durchströmenden oder an dieser Messstelle vorbeiströmenden flüssigen Mediums entspricht, und zwar einer chemischen und/oder physikalischen Eigenschaft, die u.a. auch abhängig ist von der Menge des mit dem flüssigen Medium mitgeführten Filterhilfsmittels bzw. der Konzentration dieses Filterhilfsmittels im flüssigen Medium ist.

Die von den Messstellen 12 - 14 erfassten chemischen und/oder physikalischen Werte und/oder deren Änderungen können unterschiedlichster Art sein, beispielsweise die elektrische Leitfähigkeit und/oder Kapazität und/oder Dichte und/oder Viskosität und/oder optisches Reflektionsvermögen und/oder optische Transparenz und/oder Absolutdruck und/oder Staudruck und/oder Druckdifferenz bzw. Druckabfall an einer an der betreffenden Messstelle 12 - 14 vorgesehenen Drosselblende und/oder PH-Wert usw.

Der von der Messstelle 14 gelieferte Messwert M3 entspricht somit der chemischen und/oder physikalischen Eigenschaft des Unfiltrats ohne einen Anteil an Filterhilfsmittel. Der von der Messstelle 13 gelieferte Messwert M2 entspricht den chemischen und/oder physikalischen Eigenschaften der das Filterhilfsmittel enthaltenden Suspension und der von der Messstelle 12 gelieferte Messwert den chemischen und/oder physikalischen Eigenschaften des Unfiltrats mit dem dosiert beigemischten Filterhilfsmittel.

In der Steuereinrichtung 9 bzw. in einem Rechner dieser Steuereinrichtung werden aus den Messwerten M1 - M2 unter Berücksichtigung von in einem dortigen Speicher abgelegten Kennlinien oder Kenndaten u.a. als Istwert die tatsächliche Konzentration des Filterhilfsmittels in dem den Einlass 5.1 zugeführten Unfiltrat ermittelt und mit einem in dem Speicher der Steuereinrichtung 9 ebenfalls abgelegten und/oder an einer Eingabe der Steuereinrichtung 9 eingestellten Sollwert die Dosiereinrichtung 7 so nachgeregelt bzw. nachgestellt, dass die Konzentration des Filterhilfsmittels in dem dem Einlass 5.1 zugeführten Unfiltrat möglichst exakt dem Sollwert entspricht. Dieses Nachstellen bzw. Nachregeln erfolgt beispielsweise entsprechend einer der Eigenschaften des Systems berücksichtigenden Kennlinie.

Die Verwendung von mehreren Messstellen 12 - 14 hat den Vorteil, dass durch Vergleich und/oder durch Verarbeitung der von diesen Messstellen gelieferten Messwerte M1 - M3 die durch das Zumischen des Filterhilfsmittels sich tatsächlich ergebenden Unterschiede in den chemischen und/oder physikalischen Eigenschaften des die jeweilige Messstelle 12 - 14 durchströmenden Mediums erfasst werden und dabei insbesondere auch die Möglichkeit besteht, aus den Messwerten solche Komponenten oder Anteile zu eliminieren, die aus Änderungen der chemischen und/oder physikalischen Eigenschaften des flüssigen Mediums resultieren, die nicht durch die Zugabe des Filterhilfsmittels bedingt sind.

Das Filterhilfsmittel wird der Dosiereinrichtung 7 als Suspension zugeführt, und zwar bestehend aus einem flüssigen Medium, beispielsweise aus dem Unfiltrat, in welchem das Filterhilfsmittel beispielsweise unter Verwendung eines Rührwerkes zu der Suspension aufgeschlemmt wurde.

Die Messeinrichtung 10 ist beispielsweise so ausgebildet, dass sämtliche Messstellen 12 - 14 jeweils einen Messwert M1 - M3 für eine bestimmte chemische und/oder physikalischen Eigenschaft liefern, oder aber parallel oder seriell, d.h. zeitlich aufeinander folgend mehrere Messwerte entsprechend unterschiedlichen chemischen und/oder physikalischen Eigenschaften des die jeweilige Messstelle durchströmenden flüssigen Mediums liefern, so dass durch die Erfassung unterschiedlicher chemischer und/oder physikalischer Eigenschaften die Genauigkeit der Messung und damit auch die Genauigkeit der Dosierung des Filterhilfsmittels erhöht werden kann.

Grundsätzlich besteht die Möglichkeit, dass sämtliche Messstellen 12 - 14 wenigstens ein Messsignal entsprechend der selben Art der chemischen und/oder physikalischen Eigenschaft des die Messstellen durchströmenden Mediums oder aber in jeweils einem Messzyklus mehrere Messwerte liefern, die verschiedenen Arten von chemischen und/oder physikalischen Eigenschaften des flüssigen Mediums entsprechen, wobei aber während eines jeden Messzyklus an sämtlichen Messstellen 12 - 14 jeweils die selben chemischen und/oder physikalischen Eigenschaften als Messwerte erfasst werden.

Grundsätzlich besteht weiterhin auch die Möglichkeit, die Messstellen 12 - 13 so auszubilden, dass von ihnen Messwerte M1 - M3 geliefert werden, die unterschiedlichen chemischen und/oder physikalischen Eigenschaften des flüssigen Mediums entsprechen, wobei dann diese unterschiedlichen Messwerte in der Steuereinrichtung 9 unter Berücksichtigung von dort abgelegten Kenndaten verarbeitet werden.

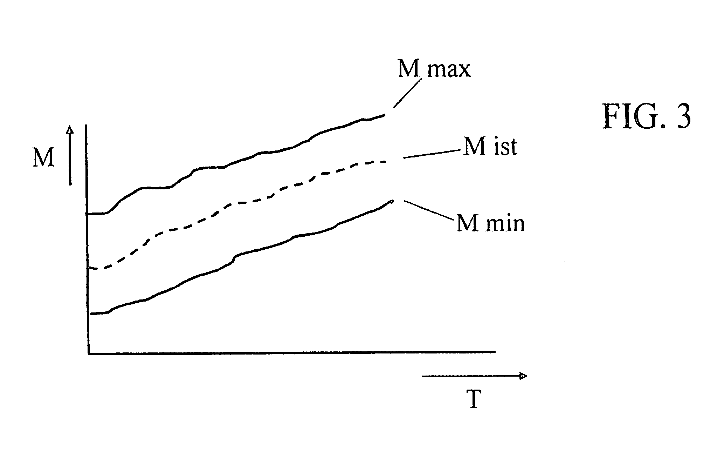

Die Figur 3 zeigt sehr schematisch einen zeitlichen Verlauf eines der Messwerte M1 l - M3. Wie in der Figur 3 dargestellt weist der jeweilige Messwert einen Maximalwert Mₘₐₓ sowie einen Minimalwert Mₘᵢₙ auf, was daraus resultiert, dass jedes Messverfahren mit bestimmten Fehlern behaftet ist. Der wahre Wert M_{IST} der Messgröße liegt dann in der Regel zwischen Mₘₐₓ und Mₘᵢₙ. Die Differenz zwischen Mₘₐₓ und Mₘᵢₙ ist von verschiedensten Faktoren abhängig, so z.B. abhängig von Fehlern des Messverfahrens, von der zu messenden Größe, von der Art des zu messenden flüssigen Mediums, von der Temperatur, von der für die Messung zur Verfügung stehenden Zeit, von dem CO2-Gehalt des flüssigen Mediums, von dem Anteil an ausgegastem, d.h. nicht mehr gelösten CO2 im flüssigen Medium usw.

Um eine möglichst genaue Dosierung des Filterhilfsmittels zu erreichen, ist die Steuereinrichtung 9 daher bevorzugt so ausgebildet, dass von den eingehenden, ein und derselben Art der chemischen und/oder physikalischen Eigenschaft entsprechenden Messwerten jeweils der tatsächliche Messwert M_{IST} gebildet wird, und zwar durch Speicherung der eingehenden Messwerte M1 - M3 und durch Berechnung des Wertes M_{IST} durch Mittelwertbildung oder nach einem anderen geeigneten Algorithmus.

Das Unfiltrat durchströmt das Rohrstück 11 in Richtung des Pfeils A. Die das wenigstens eine Filterhilfsmittel enthaltende Suspension wird dem Rohrabschnitt 11.2 in Richtung des Pfeils B zugeführt.

Die Erfindung wurde voranstehend an einem Ausführungsbeispiel beschrieben. Es versteht sich, dass Änderungen sowie Abwandlungen möglich sind, ohne dass dadurch der der Erfindung zugrunde liegende Erfindungsgedanke verlassen wird.

### Bezugszeichenliste

- 1: Anlage
- 2: Filter
- 3: Filtergehäuse
- 4: Filtratraum
- 4.1: Auslass
- 5: Unfiltratraum
- 5.1: Einlass
- 6: Filterkerze
- 7: Dosiervorrichtung
- 8: Quelle für Suspension
- 9: Steuereinrichtung
- 10: Messeinrichtung
- 11: Rohrstück
- 11.1-11.3: Rohrabschnitt
- 12, 13, 14: Messstelle
- M1, M2, M3: Messwert
- Mₘₐₓ: Maximalwert
- Mₘᵢₙ: Minimalwert
- M_{IST}: Istwert
- A: Strömungsrichtung des Unfiltrats
- B: Zuführrichtung der Suspension

## Patentansprüche

1. Verfahren zur Bestimmung oder Messung des Gehalts an wenigstens einem Filterhilfsmittel in einem flüssigen Medium, wobei mit einer ersten Messstelle (12) an wenigstens einem von dem Medium durchströmten System (11), in das das wenigstens eine Filterhilfsmittel eingebracht wird, zumindest eine chemische und/oder physikalische, durch die Zugabe des Filterhilfsmittels sich ändernde Eigenschaft des Mediums nach der Zumischung des Filterhilfsmittels gemessen wird,
**dadurch gekennzeichnet,**
**dass** mit wenigstens einer zweiten Messstelle (14) die chemische und/oder physikalische Eigenschaft des Mediums vor der Zumischung des Filterhilfsmittels gemessen wird, und dass aus den von den Messstellen (12, 14) gelieferten Messwerten (M1 - M3) die Menge des beigemischten Filterhilfsmittels ermittelt wird.

2. Verfahren nach Anspruch 1, **dadurch gekennzeichnet, dass** die chemischen und/oder physikalischen Eigenschaften des flüssigen Mediums zeitgleich an den Messstellen ermittelt werden.

3. Verfahren nach Anspruch 1 oder 2, **dadurch gekennzeichnet, dass** an wenigstens einer Messstelle (12, 13, 14) parallel oder seriell Messwerte (M1 - M3) entsprechend unterschiedlichen chemischen und/oder physikalischen Eigenschaften des flüssigen Mediums erzeugt werden.

4. Verfahren nach einem der vorhergehenden Ansprüche, **dadurch gekennzeichnet, dass** das flüssige Medium ein zu filtrierendes Unfiltrat und/oder eine das wenigstens eine Fliterhilfsmittel enthaltende Suspension ist.

5. Verfahren nach einem der vorhergehenden Ansprüche, **dadurch gekennzeichnet, dass** das Zumischen des wenigstens einen Filterhilfsmittels, beispielsweise in Form einer das wenigstens eine Filterhilfsmittel enthaltenden Suspension gesteuert und/oder geregelt unter Berücksichtigung der Messwerte (M 1 - M3) erfolgt.

6. Verfahren nach einem der vorhergehenden Ansprüche, **dadurch gekennzeichnet, dass** die mit dem Messverfahren ermittelte Menge des wenigstens einen Filterhilfsmittels aufsummiert wird.

7. Verfahren nach einem der vorhergehenden Ansprüche, **gekennzeichnet durch** die Verwendung von wenigstens drei Messstellen, von denen wenigstens zwei Messstellen (12, 14) zumindest einen den chemischen und/oder physikalischen Eigenschaften des flüssigen Mediums entsprechenden Messwert (M1, M3) und wenigstens eine weitere Messstelle (13) zumindest einen Messwert liefern, der den chemischen und/oder physikalischen Eigenschaften der das wenigstens eine Filterhilfsmittel enthaltenden Suspension entspricht.

8. Vorrichtung zur Bestimmung des Gehalts an wenigstens einem Filterhilfsmittel in einem flüssigen Medium, beispielsweise Unfiltrat, mit wenigstens einem von dem Medium durchströmbaren Kanal (11) mit wenigstens einem Anschluss (11.2) zum Zuführen des wenigstens einen beizumischenden Filtermediums, sowie mit wenigstens einer ersten Messstelle (12) zur Erzeugung wenigstens eines Messwertes (M1) in Abhängigkeit von wenigstens einer chemischen und/oder physikalischen Eigenschaft des den Kanal (11) durchströmenden das Filtermedium enthaltenden Mediums,
**gekennzeichnet**
**durch** wenigstens eine zweite und/oder dritte Messstelle (14, 13), von denen die wenigstens eine zweite Messstelle (14) wenigstens einen Messwertes (M3) in Abhängigkeit von wenigstens einer chemischen und/oder physikalischen Eigenschaft des den Kanal (11) vor dem Beimischen des Filtermediums durchströmenden Mediums und die wenigstens eine dritte Messstelle (13) wenigstens einen Messwertes (M2) in Abhängigkeit von wenigstens einer chemischen und/oder physikalischen Eigenschaft der das wenigstens eine Filterhilfsmittel enthaltenden Suspension erzeugen.

9. Vorrichtung nach Anspruch 8, **dadurch gekennzeichnet, dass** die Messstellen (12, 13, 14) für eine zeitgleiche Ermittlung der chemischen und/oder physikalischen Eigenschaften des flüssigen Mediums ausgebildet sind.

10. Vorrichtung nach Anspruch 8 oder 9, **dadurch gekennzeichnet, dass** wenigstens eine Messstelle (12, 13, 14) für eine parallel oder serielle Bildung von Messwerten (M1 - M3) entsprechend unterschiedlichen chemischen und/oder physikalischen Eigenschaften des flüssigen Mediums ausgebildet ist.

11. Vorrichtung nach einem der vorhergehenden Ansprüche, **dadurch gekennzeichnet, dass** das flüssige Medium ein zu filtrierendes Unfiltrat und/oder eine das wenigstens eine Filterhilfsmittel enthaltende Suspension ist.

12. Vorrichtung nach einem der vorhergehenden Ansprüche, **dadurch gekennzeichnet, dass** das Zumischen des wenigstens einen Filterhilfsmittels, beispielsweise in Form einer das wenigstens eine Filterhilfsmittel enthaltenden Suspension gesteuert und/oder geregelt unter Berücksichtigung der Messwerte (M1 - M3) erfolgt.

13. Vorrichtung nach einem der vorhergehenden Ansprüche, **gekennzeichnet durch** Mittel, mit denen die mit dem Messverfahren ermittelte Menge an dem wenigstens einen Filterhilfsmittel aufsummiert wird.

14. Vorrichtung nach einem der vorhergehenden Ansprüche, **dadurch gekennzeichnet, dass** der Kanal (11) wenigstens drei Abschnitte (11.1, 11.2, 11.3) aufweist, und dass an jedem Abschnitt eine Messstelle (12, 13, 14) vorgesehen ist.

## Claims

1. Method for determining or measuring the content of at least one filter aid in a liquid medium, wherein a first measuring point (12) of at least one system (11), through which the medium flows and into which the at least one filter aid is introduced, is used to measure at least one chemical and/or physical property of the medium after admixing the filter aid, said property being changed due to the addition of the filter aid,
**characterised in that**
the chemical and/or physical property of the medium prior to the addition of the filter aid is measured with at least a second measuring point (14), and that the quantity of the admixed filter aid is determined from the measured values (M1 - M3) provided by the measuring points (12, 14).

2. Method according to claim 1, **characterised in that** the chemical and/or physical properties of the liquid medium are determined simultaneously at the measuring points.

3. Method according to claim 1 or 2, **characterised in that**, at least one measuring point (12, 13, 14), measured values (M1 - M3) are generated in parallel or in series according to different chemical and/or physical properties of the liquid medium.

4. Method according to any one of the preceding claims, **characterised in that** the liquid medium is an unfiltered product to be filtered and/or a suspension which contains the at least one filter aid.

5. Method according to any one of the preceding claims, **characterised in that** the admixing of the at least one filter aid, for example in the form of a suspension containing the at least one filter aid, is carried out in a controlled and/or regulated manner taking the measured values (M1 - M3) into account.

6. Method according to any one of the preceding claims, **characterised in that** the quantity, determined with the measurement method, of the at least one filter aid is added up.

7. Method according to any one of the preceding claims, **characterised by** the use of at least three measuring points, of which at least two measuring points (12, 14) provide at least one measured value (M1, M3) which corresponds to the chemical and/or physical properties of the liquid medium and at least one further measuring point (13) provides at least one measured value which corresponds to the chemical and/or physical properties of the suspension which contains the at least one filter aid.

8. Device for determining the content of at least one filter aid in a liquid medium, for example unfiltered product, with at least one channel (11), through which the medium can flow, with at least one connection (11.2) for supplying the at least one filter medium to be admixed, and with at least a first measuring point (12) for generating at least one measured value (M1) depending on at least one chemical and/or physical property of the medium flowing through the channel (11) and containing the filter medium,
**characterised**
**by** at least one second and/or third measuring point (14, 13), of which the at least one second measuring point (14) generates at least one measured value (M3) depending on at least one chemical and/or physical property of the medium flowing through the channel (11) before the addition of the filter medium, and the at least one third measuring point (13) generates at least one measured value (M2) depending on at least one chemical and/or physical property of the suspension which contains the at least one filter aid.

9. Device according to claim 8, **characterised in that** the measuring points (12, 13, 14) are designed to simultaneously determine the chemical and/or physical properties of the liquid medium.

10. Device according to claim 8 or 9, **characterised in that** at least one measuring point (12, 13, 14) is designed to form measured values (M1 - M3) in parallel or in series according to different chemical and/or physical properties of the liquid medium.

11. Device according to any one of the preceding claims, **characterised in that** the liquid medium is an unfiltered product to be filtered and/or a suspension which contains the at least one filter aid.

12. Device according to any one of the preceding claims, **characterised in that** the admixing of the at least one filter aid, for example in the form of a suspension containing the at least one filter aid, is carried out in a controlled and/or regulated manner taking the measured values (M1 - M3) into account.

13. Device according to any one of the preceding claims, **characterised by** means with which the quantity, determined by the measurement method, of the at least one filter aid is added up.

14. Device according to any one of the preceding claims, **characterised in that** the channel (11) has at least three sections (11.1, 11.2, 11.3), and that one measuring point (12, 13, 14) is provided in each section.

## Revendications

1. Procédé servant à déterminer ou à mesurer la teneur en au moins un adjuvant de filtration dans un milieu liquide, sachant qu'on mesure après le mélange de l'adjuvant de filtration, au moins une caractéristique chimique et/ou physique du milieu, variant par l'addition de l'adjuvant de filtration, à l'aide d'un premier point de mesure (12) au niveau d'un système (11) traversé par le milieu, dans lequel système est introduit l'adjuvant de filtration au moins au nombre de un,
**caractérisé en ce**
**qu'**on mesure, avant le mélange de l'adjuvant de filtration, la caractéristique chimique et/ou physique du milieu à l'aide d'au moins un deuxième point de mesure (14), et en ce qu'on détermine à partir des valeurs de mesure (M1 - M3) fournies par les points de mesure (12, 14), la quantité de l'adjuvant de filtration ajouté.

2. Procédé selon la revendication 1, **caractérisé en ce que** les caractéristiques chimiques et/ou physiques du milieu liquide sont déterminées simultanément au niveau des points de mesure.

3. Procédé selon la revendication 1 ou 2, **caractérisé en ce que** des valeurs de mesure (M1 - M3) sont générées au niveau au moins d'un point de mesure (12, 13, 14), en parallèle ou en série, conformément aux diverses caractéristiques chimiques et/ou physiques du milieu liquide.

4. Procédé selon l'une quelconque des revendications précédentes, **caractérisé en ce que** le milieu liquide est un produit non filtré devant être filtré et/ou une suspension contenant l'adjuvant de filtration au moins au nombre de un.

5. Procédé selon l'une quelconque des revendications précédentes, **caractérisé en ce que** le mélange de l'adjuvant de filtration au moins au nombre de un, par exemple sous la forme d'une suspension contenant l'adjuvant de filtration au moins au nombre de un, est effectué de manière commandée et/ou régulée en tenant compte des valeurs de mesure (M1 - M3).

6. Procédé selon l'une quelconque des revendications précédentes, **caractérisé en ce que** la quantité déterminée par le procédé de mesure de l'adjuvant de filtration au moins au nombre de un est totalisée.

7. Procédé selon l'une quelconque des revendications précédentes, **caractérisé par** l'utilisation d'au moins trois points de mesure, parmi lesquels au moins deux points de mesure (12, 14) fournissent au moins une valeur de mesure (M1, M3) correspondant aux caractéristiques chimiques et/ou physiques du milieu liquide, et au moins un autre point de mesure (13) fournit au moins une valeur de mesure qui correspond aux caractéristiques chimiques et/ou physiques de la suspension contenant l'adjuvant de filtration au moins au nombre de un.

8. Dispositif servant à déterminer la teneur en au moins un adjuvant de filtration dans un milieu liquide, par exemple dans un produit non filtré, comprenant au moins un canal (11) pouvant être traversé par le milieu et doté d'un raccordement (11.2) servant à amener le milieu de filtration au moins au nombre de un devant être mélangé, et comprenant également au moins un premier point de mesure (12) servant à générer au moins une valeur de mesure (M1) en fonction d'au moins une caractéristique chimique et/ou physique du milieu traversant le canal (11) et contenant le milieu de filtration,
**caractérisé**
**par** au moins un deuxième et/ou un troisième point de mesure (14, 13), parmi lesquels le deuxième point de mesure (14) au moins au nombre de un génère au moins une valeur de mesure (M3) en fonction d'au moins une caractéristique chimique et/ou physique du milieu traversant le canal (11) avant le mélange du milieu de filtration et le troisième point de mesure (13) au moins au nombre de un génère au moins une valeur de mesure (M2) en fonction d'au moins une caractéristique chimique et/ou physique de la suspension contenant l'adjuvant de filtration au moins au nombre de un.

9. Dispositif selon la revendication 8, **caractérisé en ce que** les points de mesure (12, 13, 14) sont réalisés pour déterminer de manière simultanée les caractéristiques chimiques et/ou physiques du milieu fluide.

10. Dispositif selon la revendication 8 ou 9, **caractérisé en ce qu'**au moins un point de mesure (12, 13, 14) est réalisé pour former en parallèle ou en série des valeurs de mesure (M1 - M3) conformément aux diverses caractéristiques chimiques et/ou physiques du milieu fluide.

11. Dispositif selon l'une quelconque des revendications précédentes, **caractérisé en ce que** le milieu fluide est un produit non filtré devant être filtré et/ou une suspension contenant l'adjuvant de filtration au moins au nombre de un.

12. Dispositif selon l'une quelconque des revendications précédentes, **caractérisé en ce que** le mélange de l'adjuvant de filtration au moins au nombre de un, par exemple sous la forme d'une suspension contenant l'adjuvant de filtration au moins au nombre de un, est effectué de manière commandée et/ou régulée en tenant compte des valeurs de mesure (M1 - M3).

13. Dispositif selon l'une quelconque des revendications précédentes, **caractérisé par** des moyens, avec lesquels la quantité, déterminée à l'aide du procédé de mesure, de l'adjuvant de filtration au moins au nombre de un est totalisée.

14. Dispositif selon l'une quelconque des revendications précédentes, **caractérisé en ce que** le canal (11) présente au moins trois tronçons (11.1, 11.2, 11.3), et **en ce qu'**un point de mesure (12, 13, 14) est prévu au niveau de chaque tronçon.
